Europäisches Patentamt

European Patent Office

Office européen des brevets

(11) Publication number: **0 203 719**
**B1**

# EUROPEAN PATENT SPECIFICATION

(45) Date of publication of patent specification: **18.10.89**

(51) Int. Cl.⁴: **A 61 F 2/30**

(21) Application number: **86303162.1**

(22) Date of filing: **25.04.86**

(54) Implant for reconstruction of temporomandibular joint.

(30) Priority: **30.04.85 US 728820**

(43) Date of publication of application:
**03.12.86 Bulletin 86/49**

(45) Publication of the grant of the patent:
**18.10.89 Bulletin 89/42**

(84) Designated Contracting States:
**CH DE FR GB LI**

(56) References cited:
**DD-A- 226 774**
**US-A-3 178 728**
**US-A-3 579 643**
**US-A-3 992 725**

(73) Proprietor: **VITEK INC.**
**3143 Yellowstone Road**
**Houston Texas 77054 (US)**

(72) Inventor: **Homsy, Charles Albert**
**11526 Raintree Circle**
**Houston Texas 77024 (US)**
Inventor: **Tellkamp, John William**
**10034 Delmonte Drive**
**Houston Texas 77042 (US)**
Inventor: **Kent, John N.**
**5901 Wheeler**
**Metairie Louisiana 70003 (US)**

(74) Representative: **Lambert, Hugh Richmond et al**
**D. YOUNG & CO. 10 Staple Inn**
**London, WC1V 7RD (GB)**

Courier Press, Leamington Spa, England.

## Description

This invention relates, in general, to prostheses for the reconstruction of the natural temporomandibular (TM) joint, which consists of the glenoid fossa, the condyle head, the mandibular disc, and muscle attachments, and, more particularly, to improved fossa and condyle head prostheses.

. When severe functional impairment of the TM joint anatomy takes place, as from rheumatoid arthritis, osteoarthritis, tumors, and trauma, surgical TM joint reconstruction is required. Such impairment is believed to have a strong biomechanical etiology in that forces through the TM joint can become excessively large and destructive, due to abnormal muscle tension or grinding of teeth.

In U.S.A. patent 3,178,728, Christensen discloses a metallic fossa prosthesis adapted to cover or replace the natural glenoid fossa as well as the adjoining articular eminence. During the operation, to best fit within a particular patient's natural fossa and articular eminence, the surgeon had to select one from about 20 fossa prostheses of differing sizes and contours.

In U.S.A. patent 3,579,643, Morgan states that a Christensen type fossa prosthesis is needlessly oversized and may even be dangerous because it is likely (1) to disturb the normal relationship between the articular surfaces in the TM joint between the natural or artificial condyle head and the fossa prosthesis, and (2) to alter the normal bite. Morgan's proposed solution is for the prosthesis to cover only the articular eminence and not the natural fossa cavity. Morgan claims that in reducing the size of Christensen's fossa prosthesis, he requires only one right prosthesis and one left prosthesis to fit most patients.

The metallic prostheses of Christensen and of Morgan were secured by screws to the zygion. But, this fixation was found to be inadequate, because such single-sided fixation allows a fossa prosthesis to become loose due to the large loads that become exerted in the TM joint.

Previously, the present applicant has developed a tissue-ingrowth-promoting porous polymer sold under the trademark PROPLAST and which is described in detail in US—A—3,992,725. Subsequently the applicant developed a first type polymer fossa prosthesis which employed PROPLAST for postoperative permanent fixation due to tissue ingrowth. Using this fossa prosthesis and an available metallic condylar head prosthesis made in possible for a surgeon to accomplish a total reconstruction of the TM joint. This first type polymer fossa prosthesis was custom made for each patient based mostly on prior X-rays obtained from the patient's TM joint. It consisted of 3 layers: a superior layer of PROPLAST I (PTFE-graphite) implant material, a middle layer of Telfon (Trade Mark) FEP (fluorinated ethylene propylene) polymer with an embedded polyamide or metal mesh, and a most inferior layer of fused TEFLON PTFE (polytetrafluoroethylene) polymer reinforced with graphite fiber. But, this first type polymer fossa prosthesis (1) did not extend fully over the articular eminence, (2) did not adequately protect the external auditory canal, and (3) was too complex and costly to manufacture.

Subsequently, the first type polymer fossa prosthesis has been slightly modified to produce a second type fossa prosthesis which was also custom made for each patient. This slightly improved second type fossa prosthesis consisted of only 2 layers: a superior layer made of a PROPLAST II (polytetrafluoroethylene polymer with aluminium oxide) porous implant material, and of an inferior layer made of TEFLON FEP (fluorinated ethylene propylene) polymer having an embedded polyamide mesh reinforcement.

However, after using this second type implant in the reconstructed TM joint, it was found that an uneven and concentrated transfer of stresses took place between the engaging portions of the oppositely articular surfaces of the condylar head and of the fossa prostheses. Such poor load transfers can lead to great pain, bone resorption, fibrous encapsulation, loosening, extrusion, failure of the artificial fossa's material, and dislocation of the fossa and condyle prostheses within the TM joint.

It is a primary object of this invention to minimize high stress concentrations within a reconstructed TM joint. Reduction of stress concentration is critically important to the long term success of the TM joint reconstruction, because the clinical tendency of such patients is to overload the TM joint by abnormal muscle tension and/or grinding of teeth.

The surgical TM joint implant has two-parts: a first part for covering or replacing the natural glenoid fossa of a natural TM joint, and a second part for replacing the natural condylar head of the joint. The first part includes a plate having a fossa cavity defined by a relatively deep concave portion and an anteriorly located concavo-planar portion. The second part has a condylar head defining a convexo-planar, articular face for engaging the concavo-planar surface and establishing therewith a planar contact area which minimizes the unit stress transmitted between the first and second parts, thereby providing for an even load distribution between their engaged planar surfaces.

The plate is preferably shaped to fully cover the articular eminence and to protect the external acoustic meatus against external pressure.

The invention is further described in connection with the accompanying drawings, wherein:

Fig. 1 is an exploded perspective view of the preferred forms of the fossa and condyle head prostheses in accordance with the invention;

Fig. 2 is a side elevational view of a human skull carrying the artificial prostheses of Fig. 1;

Fig. 3 is a side view of the fossa prosthesis shown in Fig. 1;

Figs. 4—7 are views taken on lines 4—4 through 7—7 on Figs. 3, 4, 5 and 3, respectively;

Fig. 8 is a side plan view of the condyle head prosthesis shown in Fig. 1;

Fig. 9 is an elevational view taken on line 9—9 of Fig. 8;

Fig. 10 is a top view taken on line 10—10 of Fig. 8;

Figs. 11 and 12 show a partial side elevational view of the condyle prosthesis and a sectional view of the fossa prosthesis; Fig. 11 shows the condyle head near closed bite closure, and Fig. 12 shows the condyle head when the jaws are fully engaged;

Fig. 13 is a view taken on line 13—13 of Fig. 11;

Fig. 14 is a view taken on line 14—14 of Fig. 15, which shows applicant's above-mentioned, already-known, second type of fossa prosthesis in engagement with the prior condyle head prosthesis when it is in a position similar to that of Fig. 11;

Fig. 15 is a view taken on line 15—15 of Fig. 14;

Fig. 16 is an enlarged perspective view, similar to Fig. 2, showing the novel condyle head prosthesis in engagement with the novel fossa prosthesis;

Fig. 17 shows the planar contact surface between the novel condyle head when it is near full engagement with the novel fossa prosthesis, as shown in Fig. 11; and

Fig. 18 is a plan view of the concentrated load-transfer lines between the known condyle head prosthesis when it is in engagement with the known fossa prosthesis, as shown in Fig. 15.

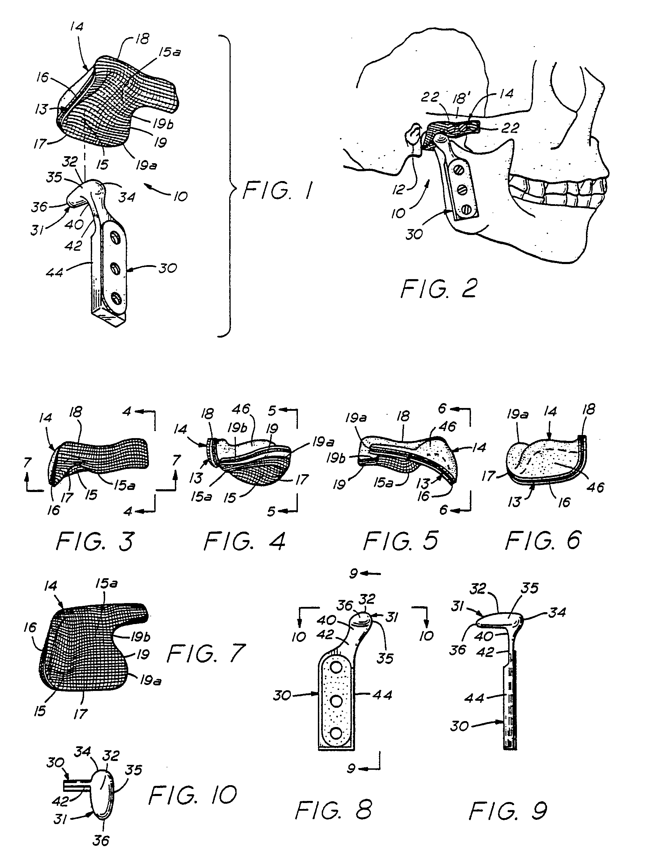

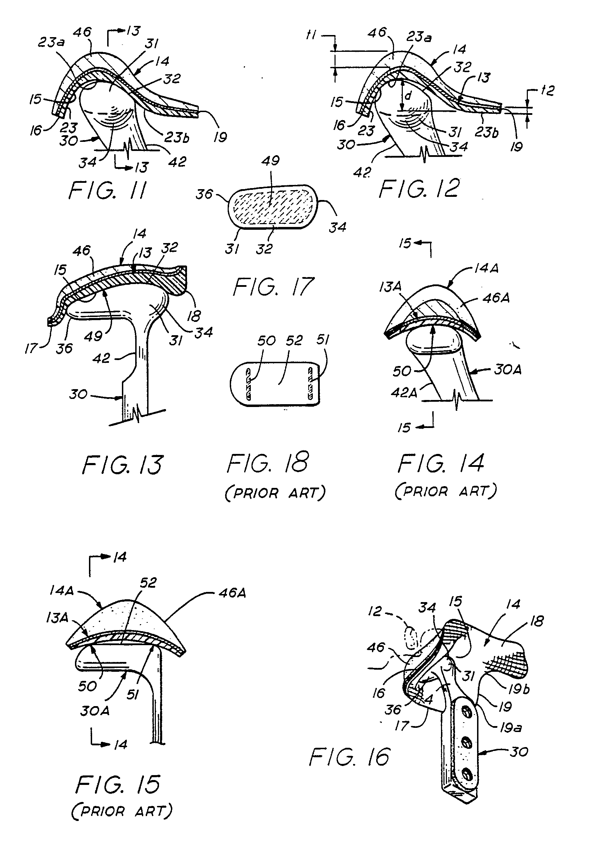

The invention will be described with general reference to Figs. 1—13 and 16—17. In Figs. 14—15 and 18, which illustrate applicant's prior fossa and condylar head prostheses, the same numerals followed by the letter "A" are used to designate corresponding parts of Figs. 1—13 and 16.

The novel composite prosthesis 10 (Figs. 1—2, 16) of this invention consists of a condyle prosthesis 30 and of a glenoid fossa prosthesis 14, which respectively constitute the ball and socket portions of a reconstructed TM joint.

The condyle head prosthesis 30 Figs. 8—9, 11—14) is made of a surgical, chrome-cobalt-molybdenum alloy and comprises a condyle head 31 which is provided with a critical load-transmitting, convexo-planar, articular face 32 (Fig. 11). A heel 34 extends backwardly and a toe 36 extends forwardly from face 32. An oval sole 35 (Figs. 1, 9) follows and extends rearwardly from face 32 and smoothly blends at its opposite ends with the heel and toe. A bridge part 40 spans between face 32, heel 34, and toe 36. A neck 42 couples bridge 40 to shank 44.

Fossa prosthesis 14 is made of a superior layer 46 of PROPLAST II (polytetrafluoroethylene polymer with aluminum oxide) porous implant material, and of an inferior plate 13 of TEFLON FEP (fluorinated ethylene propylene) polymer having an embedded polyamide mesh reinforcement therein.

Plate 13 is shaped to (1) fully cover the natural fossa cavity including the adjoining natural articular eminence, (2) give the surgeon one left fossa prosthesis and one right fossa prosthesis generally fitting the TM joints of most patients, and (3) protect the external acoustic meatus 12 (Fig. 2) against external pressure, and inhibit meatus 12 from post-operatively adhering to condyle head 31.

By using a single left and a single right fossa prostheses, the surgeon requires less operative time, while the implant manufacturer is relieved from the burden and expense of making custom fossa and condylar prostheses based on X-rays.

Plate 13 (Fig. 1) has a fossa cavity 15 which is defined by posterior rim 16, medial rim 17, lateral rim 18, and anterior rim 19. The posterior rim 16 sufficiently projects inferiorly of fossa cavity 15 to protect the external acoustic meatus 12. The protective rim 16 is moved slightly anteriorly to avoid impingement of soft tissue over the external auditory canal.

Medial rim 17 smoothly joins the posterior rim 16 and likewise projects inferiorly to cover the medial aspect of the natural glenoid fossa and the adjoining articular eminence. The anterior rim 19 has an apex 19a and projects anteriorly and obliquely superiorly to cover the articular eminence. Rim 19 is relieved at 19b to conform to the junction between the natural zygion 18' and the natural articular eminence.

Lateral rim 18 extends superiorly to and is shaped to fit along the lateral extension of zygion 18' (Fig. 2). Fossa cavity 15, anterior ridge 15a (Fig. 4—5), anterior rim 19, medial rim 17, and posterior rim 16 are shaped to offer minimum friction to the complex movements of condyle head 31 over fossa cavity 15. Rim 18 usually receives one or more screws 22 by means of which prosthesis 14 is initially attached to zygion 18', whereas the tissue-ingrowth-promoting porous PROPLAST II material provides permanent postoperative fixation for fossa prosthesis 14 within the TM joint.

There are three unique features which make the TM joint very complex: it is a non-stress and stress bearing joint, depending on the spacing between condyle head 31 and fossa cavity 15; it is a sliding hinge; and it is a bilateral joint, i.e., both the right and left sides of the joint should act in symmetry.

Therefore, fossa cavity 15 (Figs. 11—12) is provided with a stress-bearing, articular surface defined by a relatively deep concave portion 23, followed by an anteriorly located concavo-planar portion 23a, and then by a convex portion 23b.

When the TM joint is near closed bite position (Fig. 11), the articular, load-transfer, convexo-planar face 32 of condylar head 31 engages the articular, load-receiving, concavo-planar surface 23a of fossa cavity 15 and establishes therewith a relatively wide planar contact area 49 (Fig. 17) which minimizes the unit stress (kg/cm$^2$) transmitted between prostheses 14 and 30 and provides for an even load distribution between their engaged planar sliding surfaces.

In articulating the mandible from closed bite position (Fig. 12) to nearly open bite position (Fig. 11), convexo-planar, load-transfer face 32 of condyle head 31 moves forwardly on deep concave surface 23, then the planar portion of face 32 slides, while exerting very strong pressure, over the planar portion of surface 23a, and then face 32 moves over convex surface 23b.

The convexo-planar articular face 32 and the concavo-planar surface 23a are shaped to maximize the extent of the planar contact area 49 and minimize wear, as condylar head 31 translates and rotates within fossa cavity 15.

It has been unexpectedly found that minimal stress concentration at the critically near closed bite position at the mandible (Fig. 11) is provided by maximizing the extent of planar contact area 49 between the planar portion of convexo-planar face 32 and the planar portion of concavo-planar surface 23a. As an additionally benefit, fossa cavity 15 optimally distributes the significant lateral pressures to which it becomes subjected during jaw movements.

In sum, the engaging articular concavo-planar and convexo-planar surfaces 23a and 32, respectively are shaped so as to better protect the auditory canal 12 (Fig. 2) from pressure, and to more evenly and efficiently distribute the forces transmitted through the planar contact area 49, especially near closed bite position (Fig. 11), which is the critical position corresponding to maximum load transmission through the reconstructed TM joint.

This is in contrast to what one would obtain from applicant's prior glenoid fossa prosthesis 14A and condyle prosthesis 30A (Figs. 14—15). It will be readily seen that the external articular surface 52 of condyle head prosthesis 30A engages its opposite fossa plate 13A only along a pair of single-dimensional parallel lines 50 and 51 (Fig. 18), while the remainder of surface 52 between lines 50 and 51 is out of contact with plate 13A. As condyle head 30A translates and rotates over the fossa cavity defined by plate 13A, these parallel lines 50—51 also rotate, and for some positions of condyle head 30A lines 50—51 actually merge. Consequently, uneven and concentrated transfers of stress take place between condylar head prosthesis 30A and fossa prosthesis 14A. Such concentrated stresses are largest especially at near biting closure (Fig. 15) and can lead to great pain, bone resorption, fibrous encapsulation, loosening, extrusion, failure of the artificial fossa's material, and dislocation of the fossa and condyle prostheses within the TM joint.

It will be also noted that the overall size of the novel glenoid fossa prosthesis 14 is reduced as compared to prior fossa prosthesis 14A. Hence the trimming time required from the surgeon of fossa prosthesis 14 is minimized. Long term stabilization, favorable distribution of articular forces without slippage are obtained with the two-part prosthesis 10 of this invention.

In one embodiment, the maximum used thickness "t1" (Fig. 12) of the PROPLAST II coating 46 (Fig. 5) was within a range of 3—10 mm with preferred values of 4 m and 7 m, and the depth "d" of fossa cavity 15 had a range of 1 m to 9 mm with preferred values of 3 m and 6 mm. The thickness "t2" of plate 13 had a range between 1 mm and 4 mm with a preferred value of 2 mm.

From the foregoing description, it will be apparent that the invention resides primarily in the geometry of the two parts of the TM joint, not the materials used in the construction of the joint. The particular materials described, i.e. PROPLAST II, the TEFLON FEP polymer reinforced with a polyamide mesh, and the surgical, chrome-cobalt-molybdenum alloy, are merely the currently preferred materials. For a further description of PROPLAST II, reference may be had to US—A—3,992,725. TEFLON FEP is a conventional, commercially available fluorinated ethylene/propylene polymer which needs no further description

**Claims**

1. A surgical temporo-mandibular joint implant having two-parts: a first part (14) for covering or replacing the natural glenoid fossa of a natural temporo-mandibular joint, and a second part (30) for replacing the natural condylar head of said joint, wherein

said first part (14) includes a plate (13) having a fossa cavity (15) defined by a deep concave portion (23) and an anteriorly located concavo-planar portion (23a); and

said second part (30) having a condylar head (31) defining a convexo-planar, articular face (32) for engaging said concavo-planar surface (23a) and establishing therewith a planar contact area (49) which minimizes the unit stress transmitted between said first and second parts, thereby providing for an even load distribution between their engaged planar surfaces.

2. An implant according to Claim 1, characterized in that

said plate (13) is shaped to fully cover the natural fossa cavity including the adjoining natural articular eminence, and said fossa cavity (15) being followed by a convex portion (23b).

3. An implant according to claim 1 or 2, characterised in that

said head (31) has a heel (34) extending backwardly and a toe (36) extending forwardly from said face (32), an oval sole (35) following and extending rearwardly from face (32) and smoothly blending at its opposite ends with said toe and heel.

4. An implant according to claim 1, 2 or 3, characterised in that

said plate (13) is shaped to protect the external acoustic meatus (12) against external pressure.

5. An implant according to any one of claims 1—4, characterised in that the first part (14) comprises a superior layer (46) constructed of a porous, tissue ingrowth promoting material comprising polytetrafluoroethylene and aluminium oxide, and an inferior layer (13) constructed of a fluorinated ethylene/propylene polymer having a reinforcing polyamide mesh embedded therein.

**Patentansprüche**

1. Chirurgisches Temporo-Mandibula-Gelenkimplantat mit zwei Teilen: einem ersten Teil (14) zum Abdecken oder Ersetzen der natürlichen glenoiden Fossa eines natürlichen Temporo-Mandibula-Gelenkes und einem zweiten Teil (30) zum Ersetzen des natürlichen Kondylus-Kopfes des Gelenkes, wobei

das erste Teil (14) eine Platte (13) mit einer Fossa-Höhlung (15) aufweist, die durch einen tiefen konkaven Abschnitt (23) und einen davor angeordneten konkav-ebenen Abschnitt (23a) definiert ist, und

das zweite Teil (30) einen Kondylus-Kopf (31) hat, der eine konvex-ebene artikuläre Fläche (32) zum Eingriff in die konkav-ebene Fläche (23a) definiert und damit einen ebenen Berührungsbereich (49) ergibt, der die zwischen dem ersten und dem zweiten Teil übertragene einheitliche Spannung minimiert und so eine gleichmäßige Belastungsverteilung zwischen ihren aneinander angreifenden ebenen Oberflächen liefert.

2. Implantat nach Anspruch 1, dadurch gekennzeichnet, daß die Platte (13) so geformt ist, daß sie die natürliche Foss-Höhlung einschließlich der anschließenden natürlichen artikulären Erhebung abdeckt, wobei auf die Fossa-Höhlung (15) ein konvexer Abschnitt (23b) folgt.

3. Implantat nach Anspruch 1 oder 2, dadurch gekennzeichnet, daß der Kopf (31) einen sich von der Fläche (32) aus nach hinten erstreckenden Absatz (34) und einen sich nach vorn erstreckenden Fuß (36) hat, wobei eine ovale Sohle (35) folgt und sich von der Fläche (32) aus nach hinten erstreckt und sich an ihren entgegengesetzten Enden mit dem Absatz und dem Fuß glatt vermengt.

4. Implantat nach Anspruch 1, 2 oder 3, dadurch gekennzeichnet, daß die Platte (13) so geformt ist, daß sie den äußeren Gehörgang (12) gegen Außendruck schützt.

5. Implantat nach einem der Ansprüche 1 bis 4, dadurch gekennzeichnet, daß das erste Teil (14) eine obere Schicht (46), die aus einem porösen, das Gewebeeinwachsen fördernden Material besteht, welches Polytetrafluorethylen und Aluminiumoxid umfaßt, und ein untere Schicht (13), die aus einem fluorierten Ethylen/Propylen-Polymer mit darin eingebettetem Polyamidverstärkungsnetz besteht, hat.

**Revendications**

1. Implant chirurgical d'articulation temporo-mandibulaire possédant deux parties: une première partie (14) pour recouvrir ou remplacer la cavité glénoïdale naturelle d'une articulation temporomandibulaire naturelle, et une seconde partie (30) pour remplacer la tête de condyle naturelle de ladite articulation, dans lequel ladite première partie (14) comprend une plaque (13) possédant une cavité de fosse (15) délimitée par une partie concave profonde (23) et une partie concave-planaire disposée antérieurement (23a); et

ladite seconde partie (30) possédant une tête de condyle (31) délimitant une face articulaire convexe-planaire (32) pour venir en contact de ladite surface concave-planaire (23a) et établissant avec elle une surface de contact planaire (49) qui minimise la contrainte unitaire transmise entre lesdites première et seconde parties, assurant ainsi une répartition de charge uniforme entre leurs surfaces planaires en contact.

2. Implant selon la revendication 1, caractérisé en ce que ladite plaque (13) est façonnée pour recouvrir complètement la cavité de fosse naturelle y compris l'éminence articulaire naturelle adjacente, et ladite cavité de fosse (15) étant suivie d'une partie convexe (23b).

3. Implant selon la revendication 1 ou 2, caractérisé en ce que ladite tête (31) possède un talon (34) s'étendant vers l'arrière et une pointe (36) s'étendant vers l'avant depuis ladite face (32), une semelle ovale (35) suivant et s'étendant vers l'arrière depuis la face (32) et se confondant sans aspérités à ses extrémités opposées avec ladite pointe et ledit talon.

4. Implant selon la revendication 1, 2 ou 3, caractérisé en ce que ladite plaque (13) est façonnée pour protéger le conduit auditif externe (12) contre une pression externe.

5. Implant selon l'une quelconque des revendications 1 à 4, caractérisé en ce que la première partie (14) comporte une couche supérieure (46) réalisée en un matériau favorisant la croissance de tissu, poreux, comportant du polytétrafluoréthylène et de l'oxyde d'aluminium, et une couche inférieure (13) constituée d'un polymère d'éthylène/propylène fluoré possédant un treillis de polyamide de renfort noyé à l'intérieur.

FIG. 1

FIG. 2

FIG. 3

FIG. 4

FIG. 5

FIG. 6

FIG. 7

FIG. 8

FIG. 9

FIG. 10

FIG. 11

FIG. 17

FIG. 12

FIG. 13

FIG. 18
(PRIOR ART)

FIG. 14
(PRIOR ART)

FIG. 15
(PRIOR ART)

FIG. 16